(19) [European Patent Office logo] Europäisches Patentamt / European Patent Office / Office européen des brevets

(11) **EP 3 385 723 B1**

(12) **EUROPEAN PATENT SPECIFICATION**

(45) Date of publication and mention
of the grant of the patent:
**02.12.2020 Bulletin 2020/49**

(51) Int Cl.:
**G01P 5/22** *(2006.01)*    **A61B 5/026** *(2006.01)*
**A61B 5/1455** *(2006.01)*    **G01F 1/704** *(2006.01)*
**G01P 5/26** *(2006.01)*

(21) Application number: **16870320.5**

(22) Date of filing: **19.10.2016**

(86) International application number:
**PCT/JP2016/080906**

(87) International publication number:
**WO 2017/094380 (08.06.2017 Gazette 2017/23)**

(54) **INFORMATION PROCESSING DEVICE, SPECKLE IMAGING SYSTEM, AND INFORMATION PROCESSING METHOD**

INFORMATIONSVERARBEITUNGSVORRICHTUNG, SPECKLE-ABBILDUNGSSYSTEM UND INFORMATIONSVERARBEITUNGSVERFAHREN

DISPOSITIF DE TRAITEMENT D'INFORMATIONS, SYSTÈME D'IMAGERIE DE TAVELURES ET PROCÉDÉ DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2015 JP 2015238047**

(43) Date of publication of application:
**10.10.2018 Bulletin 2018/41**

(73) Proprietor: **Sony Corporation**
**Tokyo 108-0075 (JP)**

(72) Inventor: **NAKASHIMA, Yusaku**
**Tokyo 108-0075 (JP)**

(74) Representative: **D Young & Co LLP**
**120 Holborn**
**London EC1N 2DY (GB)**

(56) References cited:
**WO-A1-2010/131550**    **JP-A- H10 290 791**
**JP-A- 2014 153 154**    **US-A1- 2012 095 354**
**US-A1- 2014 316 284**    **US-A1- 2014 357 990**

- LI P ET AL: "IMAGINING CEREBRAL BLOOD FLOW THROUGH THE INTACT RAT SKULL WITH TEMPORAL LASER SPECKLE IMAGING", OPTICS LETTERS, OPTICAL SOCIETY OF AMERICA, US, vol. 31, no. 12, 15 June 2006 (2006-06-15) , pages 1824-1826, XP001243249, ISSN: 0146-9592
- Abhishek Rege ET AL: "Multiexposure laser speckle contrast imaging of the angiogenic microenvironment", journal of biomedical optics, 1 May 2011 (2011-05-01), XP055023999, DOI: 10.1117/1.3582334 Retrieved from the Internet: URL:http://scitation.aip.org/getpdf/servle t/GetPDFServlet?filetype=pdf&id=JBOPFO0000 16000005056006000001&idtype=cvips&doi=10.1 117/1.3582334&prog=normal [retrieved on 2012-04-05]

**Description**

TECHNICAL FIELD

The present disclosure relates to an information processing apparatus, a speckle imaging system, and an information processing method.

BACKGROUND ART

As a conventional method of displaying a flow rate by a speckle blood flow image, there are proposed methods including a multi-exposure speckle imaging method and the like (refer to Non-Patent Document 1).

CITATION LIST

PATENT DOCUMENT

Non-Patent Document 1: "Ashwin B. Patharathy et al.,Robust flow measurement with multi-exposure speckle imaging",Optics Express (2008)

SUMMARY OF THE INVENTION

PROBLEMS TO BE SOLVED BY THE INVENTION

According to the technique disclosed in Non-Patent Document 1, there is a need, as a prerequisite for measuring the flow rate, to perform photographing with various exposure durations in acquisition of a speckle contrast and need to provide illumination by a coherent light source having corresponding laser intensity. In order to vary the exposure duration, there is a need to adjust the intensity of the laser emission, and it is not easy to adjust the intensity of laser light emission in accordance with the exposure duration.

A dynamic range of the exposure duration of an imager can be set wider than a dynamic range of the laser intensity. While laser emission intensity is provisionally controlled at present using a powerful laser with an ND filter, or the like, this is far from efficient because a portion of the laser light is limitedly used.

The present disclosure has been made in view of the above problem, and aims to provide an information processing apparatus, a speckle imaging system, and an information processing method, capable of efficiently and easily obtaining the speckle pattern contrast as a prerequisite of measuring the fluid velocity.

SOLUTIONS TO PROBLEMS

The present inventors have intensively studied to solve the problem as described above and as a result focused on integrating the luminance of a plurality of speckle images obtained by a plurality of times of imaging using an imaging element, and has completed the present disclosure.

Specifically, the present disclosure provides an information processing apparatus including: a luminance integrator that integrates a luminance of a plurality of speckle images obtained by an imaging element by a plurality of times of imaging of scattered light obtained from an imaging target to which coherent light is emitted; and a contrast calculation unit that calculates a contrast of a speckle pattern on the basis of a speckle integrated image integrated by the luminance : integrator; wherein the contrast calculation unit calculates a variance value and an average value of the luminance in a local image region of the speckle integrated image, and calculates the contrast of the speckle pattern on the basis of the obtained variance value and average value of the luminance.

The present disclosure further provides a speckle imaging system including: the information processing apparatus according to any one of claims 1 to 11; a light source that emits coherent light on the imaging target; an imaging apparatus that performs, using an imaging element, a plurality of times of imaging of scattered light obtained from the imaging target to which the coherent light is emitted and outputs the plurality of speckle images; and a display apparatus that displays an image.

The present disclosure further provides an information processing method at least including: a luminance integration step of integrating a luminance of a plurality of speckle images obtained by an imaging element by a plurality of times of imaging of scattered light obtained from an imaging target to which coherent light is emitted; and a contrast calculation step of calculating a contrast of a speckle pattern on the basis of a speckle integrated image integrated in the luminance integration step; wherein the contrast calculation step calculates a variance value and an average value of the luminance in a local image region of the speckle integrated image, and calculates the contrast of the speckle pattern on the basis of the obtained variance value and average value of the luminance.

EFFECTS OF THE INVENTION

According to the present disclosure, it is possible to efficiently and easily obtain a contrast of a speckle pattern as a prerequisite for measuring the fluid velocity.
Note that effects described herein are non-restricting. The effects may be any effects described in the present disclosure.

BRIEF DESCRIPTION OF DRAWINGS

Fig. 1 is a schematic conceptual diagram schematically illustrating an information processing apparatus 1 according to a first embodiment of the present disclosure.
Fig. 2 is a graph illustrating a relationship between an exposure duration, speckle contrast, and a flow rate.
Fig. 3 is a flowchart illustrating an example of calculation using the information processing apparatus 1 according to the present disclosure.
Fig. 4(A) is a block diagram illustrating a wiring example of a speckle imaging system 10 according to a second embodiment of the present disclosure. Fig. 4(B) is a block diagram illustrating an internal configuration of the speckle imaging system 10 according to the second embodiment of the present disclosure.
Fig. 5 is a substitute photograph for a drawing, illustrating a concept of calculation of a speckle contrast.
Fig. 6 is a flowchart illustrating a first exemplary flow of speckle imaging using the speckle imaging system 10 according to the second embodiment of the present disclosure.
Fig. 7 is a flowchart illustrating a second exemplary flow of speckle imaging using the speckle imaging system 10 according to the second embodiment of the present disclosure.
Fig. 8 is an explanatory diagram illustrating a hardware configuration of the information processing apparatus 1 according to the first embodiment of the present disclosure.
Fig. 9 is a substitute photograph for a drawing, illustrating a map diagram obtained by mapping a blood flow rate.
Fig. 10 is a substitute photograph for a drawing, illustrating a relationship between an image captured at an exposure duration of 500 $\mu$s and the number of times of integration.
Fig. 11 is a substitute photograph for a drawing, illustrating a relationship between an image captured at an exposure duration of 500 $\mu$s and the number of times of integration.

MODE FOR CARRYING OUT THE INVENTION

Hereinafter, preferred embodiments for implementing the present disclosure will be described with reference to the drawings. The embodiment described below illustrates an exemplary representative embodiment of the present disclosure, and thus the scope of the present disclosure should not be narrowly construed. Note that description will be presented in the following order.

1. First embodiment (information processing apparatus 1)

(1) Luminance integrator 11
(2) Contrast calculation unit 12
(3) Fluid velocity calculation unit 13
(4) Display control unit
(5) Exemplary flow of calculation

2. Second embodiment (speckle imaging system 10)

(1) Light source 14
(2) Imaging apparatus 15
(3) Display apparatus 16
(4) Storage apparatus 17
(5) Imaging target O
(6) First exemplary flow of speckle imaging
(7) Second exemplary flow of speckle imaging

3. Third embodiment (information processing method)
4. Hardware configuration

<1. First embodiment (information processing apparatus 1)>

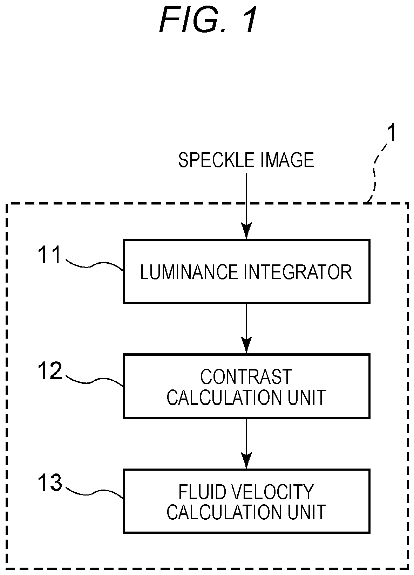

Fig. 1 is a schematic conceptual diagram schematically illustrating an information processing apparatus 1 according to a first embodiment of the present disclosure. The information processing apparatus 1 according to the present disclosure generally includes a luminance integrator 11, a contrast calculation unit 12, and a fluid velocity calculation unit 13. It is also possible to further include a display control unit and the like as necessary. Hereinafter, individual portions will be described in detail below.

(1) Luminance integrator 11

The luminance integrator 11 integrates the luminance of a plurality of speckle images obtained by an imaging element by a plurality of times of imaging of the scattered light obtained from an imaging target to which coherent light is emitted.

(2) Contrast calculation unit 12

The contrast calculation unit 12 calculates a contrast of a speckle pattern on the basis of a speckle integrated image integrated by the luminance integrator 11. In the present disclosure, the contrast of the speckle pattern is calculated as a prerequisite for measuring the blood flow in a non-invasive and noncontact manner. A portion involving the movement of a scatterer such as blood causes the speckle to change, leading to reduction in the shades of luminance. The contrast of a speckle pattern is used as an indicator of reduction in the degree of shades. A contrast K of a speckle pattern is defined by the following expression when the luminance value is I.
[Mathematical Expression 1]

$$K = \sigma/<I> \quad \cdots \quad (1)$$

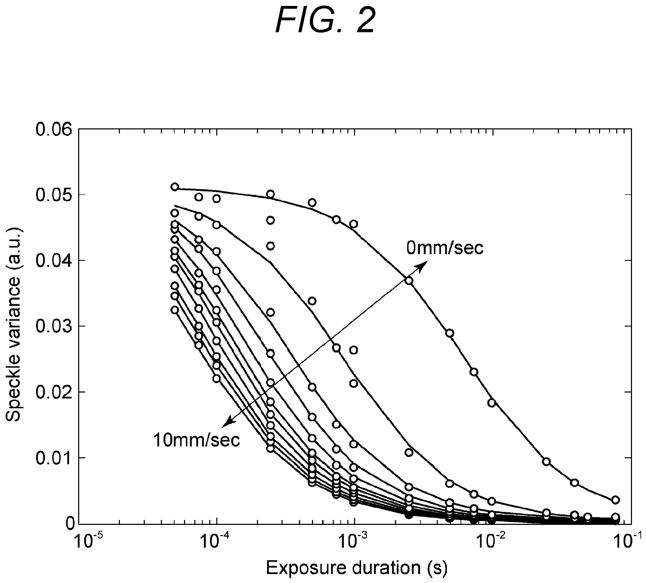

While the speckle contrast is used as an indicator of reduction in the degree of shades, this indicator is not limited to the speckle contrast.
The speckle contrast K depends on a correlation time $\tau c$ and exposure duration T. Conventionally, a multi-exposure speckle contrast, or the like, is proposed as a method for measuring the flow rate (refer to Fig. 2). Fig. 2 is a graph illustrating a relationship between the exposure duration, the speckle contrast, and the flow rate. In the conventional method (multi-exposure speckle imaging method) as illustrated in Fig. 2, there is a need to perform measurement by changing the exposure duration to various values.
The correlation time $\tau c$ is a physical quantity correlated with the flow rate and viscosity, having a small value when the flow rate is high, and a large value when the flow rate is low. As described above, there is a need to perform measurement by changing the exposure duration to various values in the conventional multi-exposure speckle imaging method in order to obtain the flow rate from the speckle contrast. In contrast, the present disclosure enables calculation of the flow rate by exposure duration patterns less than in the case of the conventional multi-exposure speckle imaging method.
The contrast calculation unit 12 according to the present disclosure calculates a variance value and an average value of the luminance in a local image region of a speckle integrated image, and calculates a speckle contrast on the basis of the obtained variance value and average value of the luminance. The contrast calculation unit 12 calculates the standard deviation of the luminance by taking a square root of the obtained variance value of the luminance and substitutes the obtained standard deviation and the average value of the luminance into the above Formula (1) to calculate the speckle contrast.

(3) Fluid velocity calculation unit 13

The fluid velocity calculation unit 13 calculates the fluid velocity of the imaging target on the basis of the integrated exposure duration obtained by integrating the exposure duration of the plurality of speckle images and the contrast of the speckle pattern calculated by the contrast calculation unit 12. An example of the fluid velocity is a blood flow rate in a blood vessel. The plurality of speckle images is images captured for an exposure duration of 10 ms or less.
Setting an upper limit value of the exposure duration to 10 ms has the following advantage. In a case where the blood flow is imaged for an exposure duration of about 15 ms, the speckle contrast (speckle variance) becomes smaller at about 1 mm/sec. Therefore, even when the speckle contrast is integrated, the value would not change. This is because the variance of speckle has already decreased at the exposure duration of 10 ms at the flow rate of the blood flow to be measured, making it difficult to take an advantage of integration.

With reference to the graph of Fig. 2, speckle variance values of individual flow rates have not sufficiently reduced in the case of $10^{-3}$sec and $10^{-4}$sec, and thus, integration of the speckle contrasts takes effects.

In contrast, when the value increased to exceed $10^{-2}$ sec, the speckle variance values fall to the bottom, making it difficult to allow the integration to take effect. Consequently, it is preferable to set the exposure duration to about $10^{-3}$ sec or less, or about $10^{-4}$ sec.

As described above, the fluid velocity calculation unit 13 calculates the blood flow rate using the integrated exposure duration. Typically, flow rate sensitivity of the blood flow differs in dependence on the length of exposure duration. With an image obtained by averaging the speckle images captured with the exposure duration so short that the blood movement is not visible during exposure, it is possible to obtain the blood flow rate in the corresponding region. For example, with the image obtained by averaging the images in the number of 2,..., 10,..., 100,..., 1000,..., it is possible to obtain an image equivalent to the image captured with twice, 10 times,., 100 times,..., 1000 times,... the exposure duration.

The fluid velocity calculation unit 13 obtains a correlation time τc by substituting the contrast K of a local speckle pattern of the obtained image and the integrated exposure duration T into the following Formula (2) .

[Mathematical Expression 2]

$$K(T, \tau_c) = \left( \frac{1 - e^{-2\frac{T}{\tau_c}}}{2\frac{T}{\tau_c}} \right)^{\frac{1}{2}} \qquad \cdots \ (2)$$

where K (T, $\tau_c$) is the contrast of the speckle pattern, T is the exposure duration, and $\tau_c$ is the correlation time.

The fluid velocity calculation unit 13 obtains a correlation time on the basis of the integrated exposure duration and the speckle contrast, and then, compares the obtained correlation time with a predetermined correlation time to calculate the blood flow rate. Note that the predetermined correlation time is a correlation time obtained from the calibration curve or the correlation time obtained in advance. Note that in a case where the correlation time is not measured in advance, a relative flow rate can be obtained by directly comparing the correlation time obtained in a plot.

In addition to the above Formula (2), it is possible to use the following Formula (3) indicating the relationship between the exposure duration, the speckle contrast, and the correlation time. The value determined in plotting the blood flow at a known flow rate can be used for β in Formula (3).

[Mathematical Expression 3]

$$K(T, \tau_c) = \left( \beta \frac{e^{-2\frac{T}{\tau_c}} - 1 + 2\frac{T}{\tau_c}}{2\left(\frac{T}{\tau_c}\right)^2} \right)^{\frac{1}{2}} \qquad \cdots \ (3)$$

where K (T, $\tau_c$) is the contrast of the speckle pattern, T is the exposure duration, $\tau_c$ is the correlation time, and β is a value determined when a known blood flow rate is plotted.

(4) Display control unit

The display control unit controls the display unit to display an image. The display control unit can map the fluid velocity calculated by the fluid velocity calculation unit 13 to further control the display unit to display fluid velocity distribution.

(5) Exemplary flow of calculation

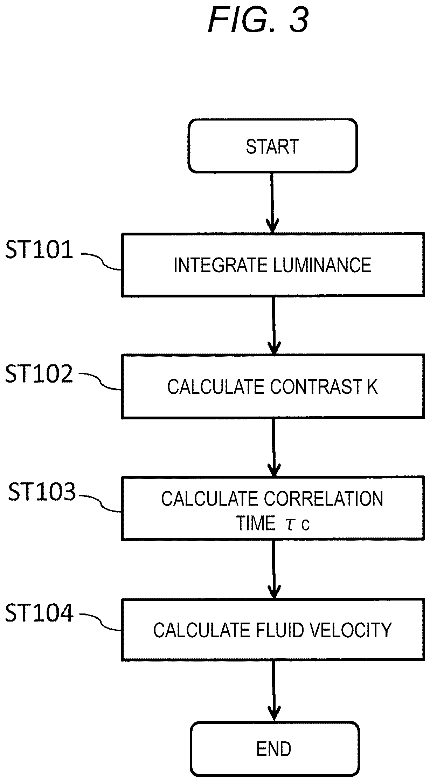

Fig. 3 is a flowchart illustrating an example of calculation using the information processing apparatus 1 according to the present disclosure. Hereinafter, exemplary flows will be described along the time series.

(a) Integration of luminance

First, in step ST101, the luminance integrator 11 integrates the luminance of a plurality of speckle images.

(b) Calculation of contrast of speckle pattern

Next, in step ST102, the contrast calculation unit 12 calculates the contrast K of the speckle pattern on the basis of the speckle integrated image integrated by the luminance integrator 11.

(c) Calculation of correlation time

Subsequently, the fluid velocity calculation unit 13 calculates the fluid velocity of the imaging target on the basis of the integrated exposure duration T and the contrast K of the speckle pattern. In step ST103, the fluid velocity calculation unit 13 calculates the correlation time $\tau c$ on the basis of the integrated exposure duration T and the contrast K. Specifically, the integrated exposure duration T and the contrast K are substituted into the above Formula (2) to calculate the correlation time $\tau c$.

(d) Calculation of fluid velocity

Then, in step ST104, the fluid velocity calculation unit 13 compares the calculated correlation time $\tau c$ with a predetermined correlation time $\tau c$ to calculate the fluid velocity.

<2. Second embodiment (speckle imaging system 10)>

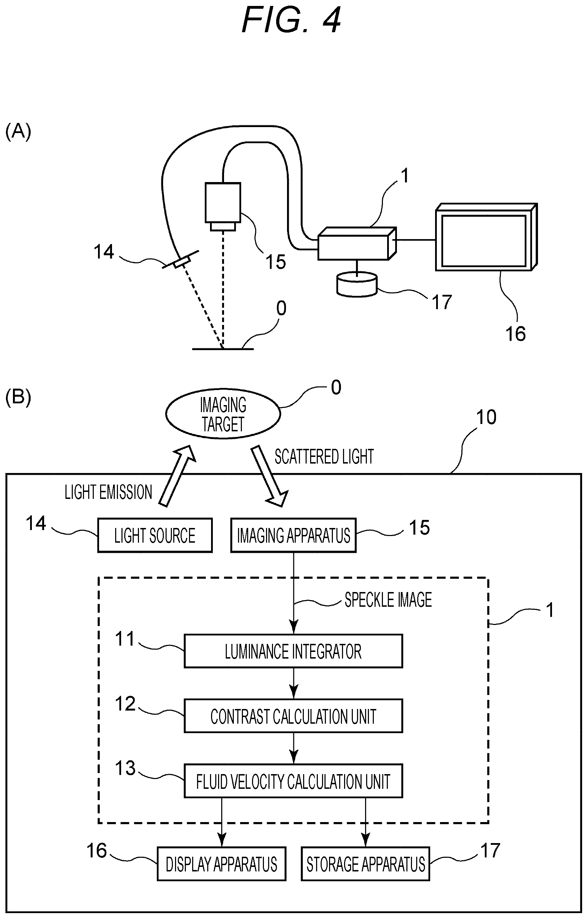

Fig. 4(A) is a block diagram illustrating a wiring example of a speckle imaging system 10 according to a second embodiment of the present disclosure. Fig. 4(B) is a block diagram illustrating an internal configuration of the speckle imaging system 10 according to the second embodiment of the present disclosure. Note that in Figs. 4(A) and 4(B), the same reference numerals are given to the same components as those of the information processing apparatus 1 according to the present disclosure, and the detailed description thereof will be omitted. The speckle imaging system 10 according to the present disclosure generally includes the information processing apparatus 1, a light source 14, an imaging apparatus 15, and a display apparatus 16. It is also possible to further include a storage apparatus 17 and the like as necessary.

(1) Light source 14

The light source 14 emits coherent light on an imaging target O. Coherent light represents light in which the phase relationship of the light waves at arbitrary two points in a light flux is temporally invariable and constant and exhibiting complete coherence after the light flux is first split by an arbitrary method then combined again with a great light path difference.
The types of the light source of the coherent light emitted by the light source 14 is not particularly limited as long as the effect of the present technology is not impaired. An example of the coherent light is laser light. Examples of the light source 14 that emits laser light include an argon ion (Ar) laser, a helium-neon (He-Ne) laser, a dye laser, a krypton (Cr) laser, a semiconductor laser, and a solid-state laser combining the semiconductor laser with wavelength conversion optical elements, or any free combination of the one or two of the above.

(2) Imaging apparatus 15

Using the imaging element, the imaging apparatus 15 performs a plurality of times of imaging of the scattered light obtained from the imaging target O to which the coherent light is emitted, and outputs a plurality of speckle images.

The imaging method used by the imaging apparatus 15 is not particularly limited as long as the effect of the present technology is not impaired, and one or two or more known imaging methods can be combined and used in an arbitrary manner. For example, it is allowable to use an imaging method using an imaging element such as a charge coupled device (CCD) or a complementary metal oxide semiconductor (CMOS) sensor.

In the present disclosure, the exposure duration of the imaging apparatus 15 is set to 10 ms or less, and the duration is preferably set to 1 ms or less, and more preferably set to about 100 μs.

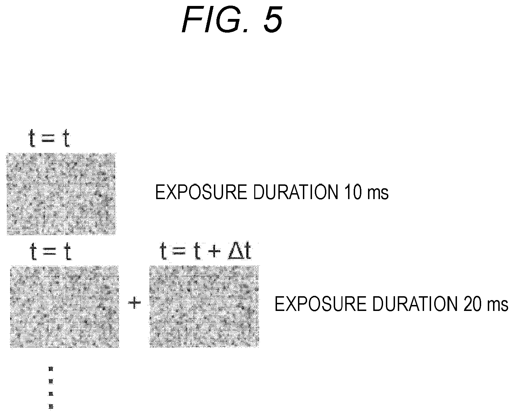

As a specific method in the present disclosure, a coherent light source such as a laser is used to illuminate a subject (such as a living body) to obtain a plurality of images (two or more images) using an imaging element such as a CCD or a CMOS with a short exposure duration of 10 ms or below. The exposure duration may be the exposure duration that would not cause a decrease in the speckle contrast due to the movement of the scatterer. In this case, a plurality of images are averaged to achieve an effect of extending the exposure duration. In a case where an optimum value of the exposure duration is unknown, performing the photographing with sufficiently short exposure duration enables adjustment of the exposure duration by post-processing. For example, two frames of an image captured with a fixed exposure duration (10 ms) can be added to obtain an image equivalent to 20 ms, or three frames of the image can be added to obtain an image equivalent to 30 ms (refer to Fig. 5). Fig. 5 is a substitute photograph for a drawing, illustrating a concept of calculation of the speckle contrast. The integrated exposure duration (20 ms or 30 ms) obtained by mathematical processing of adding two or three frames of a certain exposure duration (10 ms) is used for calculating the blood flow rate in the fluid velocity calculation unit 13.

(3) Display apparatus 16

The display apparatus 16 displays an image such as a speckle integrated image integrated by the luminance integrator 11. Moreover, the display apparatus 16 further enables the blood flow rate calculated by the fluid velocity calculation unit 13 to be mapped to further display the distribution of the blood flow rate.

(4) Storage apparatus 17

The storage apparatus 17 stores the speckle integrated image integrated by the luminance integrator 11, the speckle contrast K calculated by the contrast calculation unit 12, or the like. Moreover, the storage apparatus 17 can further store the distribution of the blood flow rate.

(5) Imaging target O

The speckle imaging system 10 according to the present disclosure can set various types of objects as the imaging target, and thus can be suitably used in, for example, imaging that sets the object containing a fluid as the imaging target. More specifically, a living body may be set as the imaging target O, and blood may be used as a fluid. For example, with application of the speckle imaging system 10 according to the present disclosure to a surgical microscope, a surgical endoscope, or the like, it is possible to perform surgery while confirming the position of blood vessels. This makes it possible to achieve safer and highly accurate surgeries, and contribute to further development of medical technology.

(6) First exemplary flow of speckle imaging

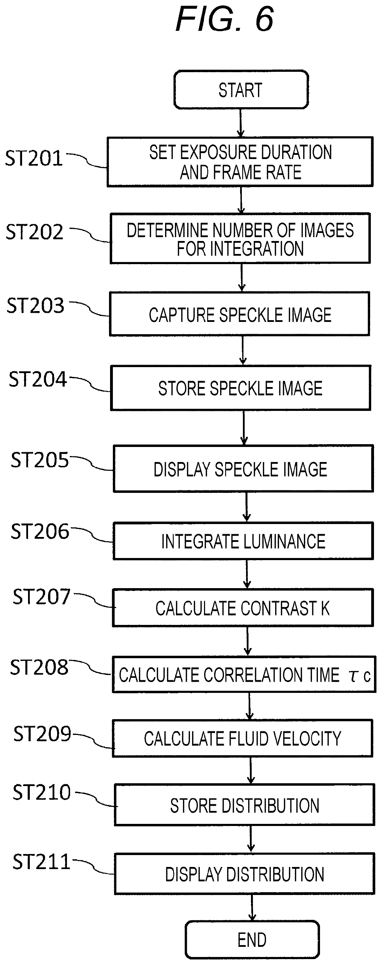

Fig. 6 is a flowchart illustrating a first exemplary flow of speckle imaging using the speckle imaging system 10 according to the second embodiment of the present disclosure. In the first exemplary flow, the correlation time τc measured in advance is compared with the correlation time τc obtained by the fluid velocity calculation unit 13 so as to calculate the blood flow rate. Hereinafter, the first exemplary flow will be described along the time series.

(a) Setting of exposure duration and frame rate

First, in step ST201, the exposure duration and the frame rate are set in the imaging apparatus 15.

(b) Determination of total number of images for integration

Next, in step ST202, on the imaging apparatus 15, the number of images for integration, specifically, for which the luminance is to be integrated is determined on the basis of the exposure duration and the frame rate set in step ST201.

(c) Image of speckle image

Next, in step ST203, the imaging apparatus 15 uses the imaging element to perform imaging the number of times equivalent to the integrated number determined in step ST202 using the imaging element so as to output a plurality of speckle images to the information processing apparatus 1.

(d) Storing speckle images

Next, in step ST204, the plurality of speckle images is recorded in the storage apparatus 17.

(e) Displaying speckle images

Next, in step ST205, the plurality of speckle images is displayed on the display apparatus 16.
Note that it is allowable to execute any one of the above steps ST204 and ST205. In addition, speckle image storage processing (step ST204) may be performed after speckle image display processing (step ST205). Note that the speckle image is stored in order to analyze the speckle image later. In addition, the speckle image is displayed in order to confirm the photographed speckle image.

(f) Integration of luminance

Next, in step ST206, the luminance integrator 11 of the information processing apparatus 1 integrates the luminance of a plurality of speckle images.

(g) Calculation of contrast of speckle pattern

Next, in step ST207, the contrast calculation unit 12 of the information processing apparatus 1 calculates the contrast K of the speckle pattern on the basis of the speckle integrated image integrated by the luminance integrator 11. Note that the contrast K may be stored in the storage apparatus 17.

(h) Calculation of correlation time

Subsequently, the fluid velocity calculation unit 13 of the information processing apparatus 1 calculates the fluid velocity of the imaging target on the basis of the integrated exposure duration T and the contrast K of the speckle pattern. In step ST208, the fluid velocity calculation unit 13 calculates the correlation time $\tau c$ on the basis of the integrated exposure duration T and the contrast K. Specifically, the integrated exposure duration T and the contrast K are substituted into the above Formula (2) to calculate the correlation time $\tau c$. Note that the correlation time $\tau c$ may be stored in the storage apparatus 17.

(i) Calculation of fluid velocity

In step ST209, the fluid velocity calculation unit 13 compares the calculated correlation time $\tau c$ with a predetermined correlation time $\tau c$ to calculate the fluid velocity. Note that the contrast K may be stored in the storage apparatus 17 and may be displayed on the display apparatus 16.

(j) Storing fluid velocity distribution

Next, in step ST210, a fluid velocity distribution obtained by mapping the fluid velocities calculated by the fluid velocity calculation unit 13 is stored in the storage apparatus 17.
Then, in step ST211, the fluid velocity distribution obtained by the mapping is displayed on the display apparatus 16.

(7) Second exemplary flow of speckle imaging

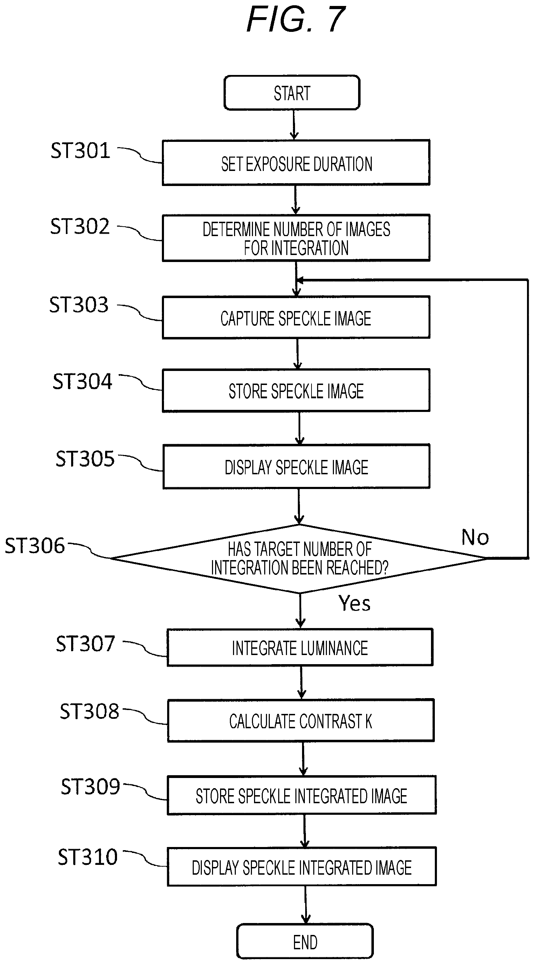

Fig. 7 is a flowchart illustrating a second exemplary flow of speckle imaging using the speckle imaging system 10 according to the second embodiment of the present disclosure. In this second exemplary flow, imaging of the speckle image is iteratively executed until the target number of sheets to undergo luminance integration is reached. Hereinafter, the second exemplary flow will be described along the time series.

(a) Setting of exposure duration

First, in step ST301, the exposure duration is set in the imaging apparatus 15.

(b) Determination of total number of images for integration

Next, in step ST302, on the imaging apparatus 15, the number of images for integration, specifically, for which the luminance is to be integrated is determined on the basis of the exposure duration set in step ST301.

(c) Image of speckle image

Next, in step ST303, the imaging apparatus 15 performs imaging using the imaging element and outputs the speckle image to the information processing apparatus 1.

(d) Storing speckle images

Next, in step ST304, a plurality of speckle images is stored in the storage apparatus 17. Note that the storage apparatus 17 may store the number of times of imaging (total number of integrated images).

(e) Displaying speckle images

Next, in step ST305, a speckle image is displayed on the display apparatus 16.
Note that it is allowable to execute any one of the above steps ST304 and ST305. In addition, speckle image storage processing (step ST304) may be performed after speckle image display processing (step ST305). Note that the speckle image is stored in order to analyze the speckle image later. In addition, the speckle image is displayed in order to confirm the photographed speckle image.

(f) Counting number of sheets integrated

Next, in step ST306, the imaging apparatus 15 counts the number of times of imaging (integrated number of images). In a case where the target number of times of imaging has not been reached, the processing returns to step ST303 and imaging is repeated. In a case where the number of times of imaging has reached the target number of times, the processing proceeds to step ST307.

(g) Integration of luminance

Next, in step ST307, the luminance integrator 11 of the information processing apparatus 1 integrates the luminance of a plurality of speckle images.

(h) Speckle pattern contrast calculation

Next, the contrast calculation unit 12 of the information processing apparatus 1 calculates, in step ST308, the contrast K of the speckle pattern on the basis of the speckle integrated image integrated by the luminance integrator 11. Note that the contrast K may be stored in the storage apparatus 17.

(i) Storing speckle integrated image

Next, a speckle integrated image is stored in the storage apparatus 17 in step ST309.

(j) Displaying speckle integrated image

Then, in step ST310, a speckle integrated image is displayed on the display apparatus 16.

Note that it is allowable to execute any one of the above steps ST309 and ST310. In addition, speckle image storage processing (step ST309) may be performed after speckle image display processing (step ST310).

3. Third embodiment (information processing method)>

The information processing method according to the present disclosure generally includes steps of at least a luminance integration step and a speckle contrast calculation step. It is also possible to further perform a fluid velocity calculation step, a storage step, a display step, or the like, as necessary. Note that the luminance integration step, the speckle contrast calculation step, the fluid velocity calculation step, the display step, and the storage step are the same as the method respectively performed by the luminance integrator 11, the contrast calculation unit 12, the fluid velocity calculation unit 13, the display apparatus 16, and the storage apparatus 17, of the speckle imaging system 10 according to the present disclosure described above, and thus, description is omitted here.

<4. Hardware configuration>

Processing of the information processing apparatus 1 according to the first embodiment described above is implemented by cooperation of software and the hardware described below.

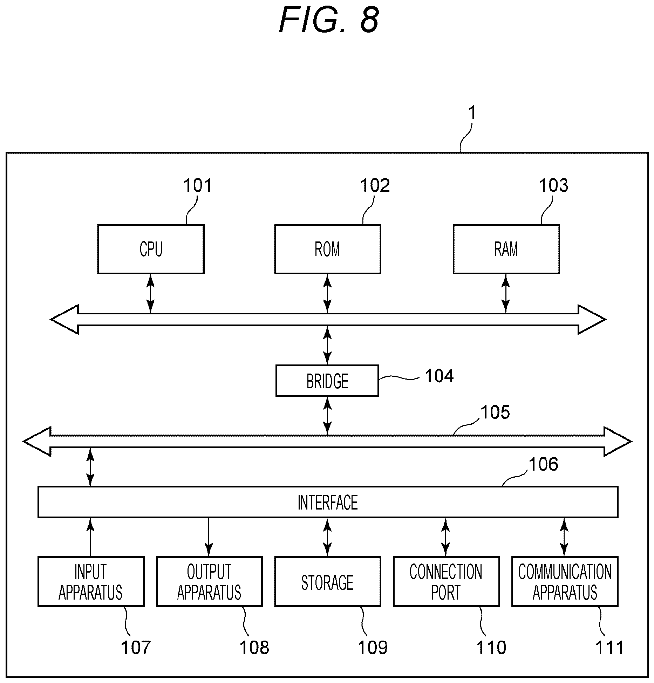

Fig. 8 is an explanatory diagram illustrating a hardware configuration of the information processing apparatus 1 according to the first embodiment of the present disclosure. As illustrated in Fig. 8, the information processing apparatus 1 includes a central processing unit (CPU) 101, a read only memory (ROM) 102, a random access memory (RAM) 103, a bridge 104, a bus 105, an interface 106, an input apparatus 107, an output apparatus 108, a storage 109, a connection port 110, and a communication apparatus 111.

The CPU 101 functions as an information processing apparatus and cooperates with various programs to implement operation of the luminance integrator 11, the contrast calculation unit 12 and the fluid velocity calculation unit 13 in the information processing apparatus 1. Moreover, the CPU 101 may be a microprocessor. The ROM 102 stores programs, calculation parameters, or the like, used by the CPU 101. The RAM 103 temporarily stores programs to be used in the execution by the CPU 101 or parameters, or the like, appropriately changing in execution. A portion of the memory in the information processing apparatus 1 is implemented by the ROM 102 and the RAM 103. The CPU 101, the ROM 102, and the RAM 103 are mutually connected by an internal bus including a CPU bus and the like.

The input apparatus 107 includes an input apparatus used to input information by a user, such as a touch screen, a button, a microphone, a switch, and a lever, and an input control circuit and the like that generates an input signal on the basis of the input by the user and outputs the signal to the CPU 101. The user of the information processing apparatus 1 operates the input apparatus 107 to enable inputting various types of data or instructing processing operation to the information processing apparatus 1.

The output apparatus 108 performs outputs to an apparatus such as a liquid crystal display (LCD) device, an organic light emitting diode (OLED) device, and a lamp. Moreover, the output apparatus 108 may output sounds using a speaker, a headphone, and the like from the viewpoint of user friendliness.

The storage 109 is an apparatus for storing data. From the viewpoint of user friendliness, the storage 109 may include a storage medium, a recording apparatus that records data in the storage medium, a reading apparatus that reads data from the storage medium, a deletion apparatus that deletes data recorded in the storage medium, and the like. The storage 109 stores programs executed by the CPU 101 and various data.

The connection port 110 is a bus used to connect with an external apparatus or a peripheral apparatus, for example, from the information processing apparatus 1. In addition, from the viewpoint of user friendliness, the connection port 110 may be a universal serial bus (USB) type.

The communication apparatus 111 is, for example, a communication interface including communication devices for connecting to a network. In addition, from the viewpoint of user friendliness, the communication apparatus 111 may be an infrared communication compatible apparatus, a wireless local area network (LAN) compatible communication apparatus, a long term evolution (LTE) compatible communication apparatus, or may be a wired communication apparatus performing wired communication.

As described above, according to the first to third embodiments, it is possible to efficiently and easily obtain a contrast of a speckle pattern as a prerequisite for measuring the fluid velocity.

Moreover, it is possible in the first to third embodiments to perform imaging with a constant exposure duration without changing the intensity of the coherent light source (with constant intensity). This makes it possible to measure the blood flow rate with a simplified apparatus without a need to provide external control for attenuating the laser, leading to the reduction in cost and technical difficulty.

Moreover, it is possible to obtain an image in which the blood flow rates are mapped in the first to third embodiments. In conventional proposed methods of displaying the flow rate by speckle contrast including the multi-exposure speckle imaging method and the like, there is a need to perform imaging at various exposure durations and thus need illumination by a coherent light source having the corresponding intensity. While the exposure duration can be easily adjusted, it was difficult to widely change the illumination intensity of the coherent light source. The first to third embodiments described above enables calculation of the blood flow rate with the number of exposure duration patterns less than the case of the conventional multi-exposure speckle imaging method. Accordingly, measurement of the blood flow rate can be implemented with a simplified apparatus without a need to provide external control to widely change the illumination intensity of the coherent light source as in the conventional multi-exposure speckle imaging, leading to the reduction in cost and technical difficulty.

To perform observation of the movement (flow) of a scatterer using speckle contrast, there is a need to extend the exposure duration to some extent. For example, in the case of using a digital imager, there is a need to allow the scatterer to move by one pixel or more (to the extent to change the speckle pattern) during the exposure duration. With the conventional method, it is difficult to calculate the speckle contrast unless an optimum exposure duration for the measurement target is set as the exposure duration. As in the present embodiment, while the exposure duration can be adjusted by post-processing, it is impractical to shorten the exposure duration by post-processing. In order to set the optimum exposure duration by post-processing, there is a need to achieve shorter exposure duration.

Moreover, in the first to third embodiments, it is possible to optimize the exposure duration after photographing in order to calculate the speckle contrast. Furthermore, it is also possible to depict velocities of flows in states such as low or still (but with Brownian motion) not depictable in a speckle blood flow image (that is, the dynamic range of flow rate sensitivity can be expanded).

Herein, optimization represents performing blood flow imaging optimal for the following applications (A) to (C), for example. That is, it is possible to obtain an optimum blood flow image corresponding to the blood flow of the blood vessel in performing (A) detecting a portion of normal blood flow and a portion of abnormal blood flow, (B) detecting a portion of poor blood flow due to a certain blockage even without stenosis (portion of poor blood flow) in the blood vessel, and (C) implementation of stenosis level evaluation (to determine how many overlapped images are needed to find a stenosis, etc.).

In addition, the present disclosure can be configured as follows.

(1) An information processing apparatus including:

a luminance integrator that integrates a luminance of a plurality of speckle images obtained by an imaging element by a plurality of times of imaging of scattered light obtained from an imaging target to which coherent light is emitted; and
a contrast calculation unit that calculates a contrast of a speckle pattern on the basis of a speckle integrated image integrated by the luminance integrator.

(2) The information processing apparatus according to (1), further including a fluid velocity calculation unit that calculates a fluid velocity of the imaging target on the basis of an integrated exposure duration obtained by integrating exposure durations of the plurality of speckle images and the contrast of the speckle pattern calculated by the contrast calculation unit.
(3) The information processing apparatus according to (1) or (2), in which the plurality of speckle images is images captured for an exposure duration of 10 ms or less.
(4) The information processing apparatus according to any of (1) to (3),
in which the contrast calculation unit calculates a variance value and an average value of the luminance in a local image region of the speckle integrated image, and calculates the contrast of the speckle pattern on the basis of the obtained variance value and average value of the luminance.
(5) The information processing apparatus according to (4),
in which the contrast calculation unit calculates a standard deviation of the luminance by taking a square root of the obtained variance value of the luminance and substitutes the obtained standard deviation and the average value of the luminance into Formula (1) indicating a relationship between the contrast of the speckle pattern, the standard deviation of the luminance, and the average value of the luminance so as to calculate the contrast of the speckle pattern,
[Mathematical Expression 1]

$$K = \sigma / <I> \quad \cdots \quad (1)$$

where K is the contrast of the speckle pattern, $\sigma$ is the standard deviation of luminance I, and <I> is the average value of luminance I.

(6) The information processing apparatus according to (2),

in which the fluid velocity calculation unit obtains a correlation time on the basis of the integrated exposure duration and the contrast of the speckle pattern, and compares the obtained correlation time with a predetermined correlation time to calculate the fluid velocity.

(7) The information processing apparatus according to (6),

in which the fluid velocity calculation unit substitutes the integrated exposure duration and the contrast of the speckle pattern into Formula (2) indicating a relationship between the exposure duration, the contrast of the speckle pattern, and the correlation time so as to obtain the correlation time,

[Mathematical Expression 2]

$$K(T, \tau_c) = \left( \frac{1 - e^{-2\frac{T}{\tau_c}}}{2\frac{T}{\tau_c}} \right)^{\frac{1}{2}} \qquad \dots \ (2)$$

where $K(T, \tau_c)$ is the contrast of the speckle pattern, T is the exposure duration, and $\tau_c$ is the correlation time.

(8) The information processing apparatus according to (6),

in which the fluid velocity calculation unit substitutes the integrated exposure duration and the contrast of the speckle pattern into Formula (3) indicating the relationship between the exposure duration, the contrast of the speckle pattern, and the correlation time so as to obtain the correlation time,

[Mathematical Expression 3]

$$K(T, \tau_c) = \left( \beta \frac{e^{-2\frac{T}{\tau_c}} - 1 + 2\frac{T}{\tau_c}}{2\left(\frac{T}{\tau_c}\right)^2} \right)^{\frac{1}{2}} \qquad \dots \ (3)$$

where $K(T, \tau_c)$ is the contrast of the speckle pattern, T is the exposure duration, $\tau_c$ is the correlation time, and $\beta$ is a value determined when a known blood flow rate is plotted.

(9) The information processing apparatus according to (2), further including a display control unit that controls a display unit to display an image.

(10) The information processing apparatus according to (9), in which the display control unit maps the fluid velocity calculated by the fluid velocity calculation unit to further control the display unit to display fluid velocity distribution.

(11) The information processing apparatus according to (2), in which the fluid velocity is a blood flow rate in a blood vessel.

(12) A speckle imaging system including:

the information processing apparatus according to any of (1) to (11);
a light source that emits coherent light to an imaging target;
an imaging apparatus that performs, using an imaging element, a plurality of times of imaging of scattered light from the imaging target to which the coherent light is emitted and outputs the plurality of speckle images; and
a display apparatus that displays an image.

(13) The speckle imaging system according to (12), in which the display apparatus maps the fluid velocity calculated by the fluid velocity calculation unit to further display fluid velocity distribution.

(14) An information processing method at least including:

a luminance integration step of integrating a luminance of a plurality of speckle images obtained by an imaging element by a plurality of times of imaging of scattered light obtained from an imaging target to which coherent light is emitted; and

a contrast calculation step of calculating a contrast of a speckle pattern on the basis of a speckle integrated image integrated in the luminance integration step.

Note that effects described here in the present specification are provided for purposes of exemplary illustration and are not intended to be limiting. Still other effects may also be contemplated.

Experimental examples

Hereinafter, effects of the present disclosure will be described specifically by giving experimental examples of the present disclosure.

<First experimental example>

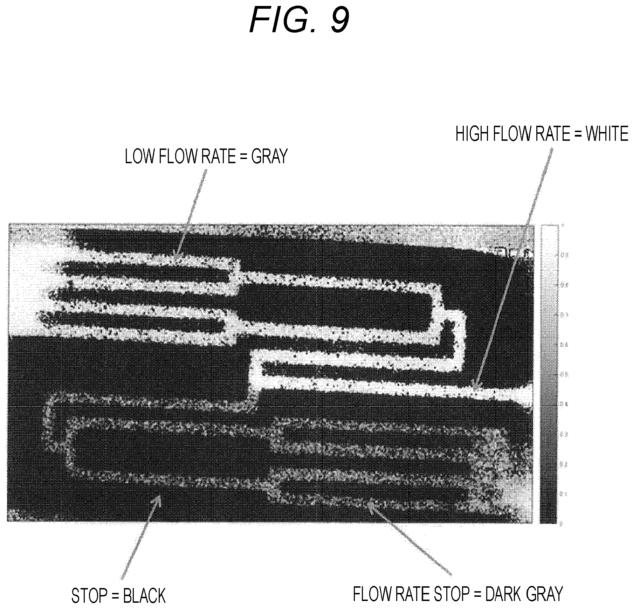

In a first experimental example, a living body uniformly illuminated by a coherent laser light source with a wavelength of 820 nm was imaged at a frame rate of 120 fps and an exposure duration of 100 μs using a SONY global shutter CMOS imager. Note that the luminance I is obtained by the SONY global shutter CMOS imager. Speckle images in the number of one, 10, 100, and 1000 in time series were integrated to obtain speckle integrated images corresponding to the exposure durations of 100 μs, 1 ms, 10 ms, and 100 ms. The above Formula (1) was used to obtain the local speckle contrast K on the basis of each of the speckle integrated images. The speckle contrast K and the integrated exposure duration T were plotted to be fitted by the above Formula (2) to calculate the correlation time τc. The correlation time τc obtained by the calculation was compared with the correlation time τc calculated by the known flow rate to obtain the blood flow rate at a local site, and the obtained blood flow rate is mapped (refer to Fig. 9). Fig. 9 is a substitute photograph for a drawing, illustrating a map diagram obtained by mapping the blood flow rate. The color of the map diagram can be used to detect the portion where the blood flow is normal and the portion where the blood flow is not normal, and grasp the blood flow rate.

<Second Experimental Example>

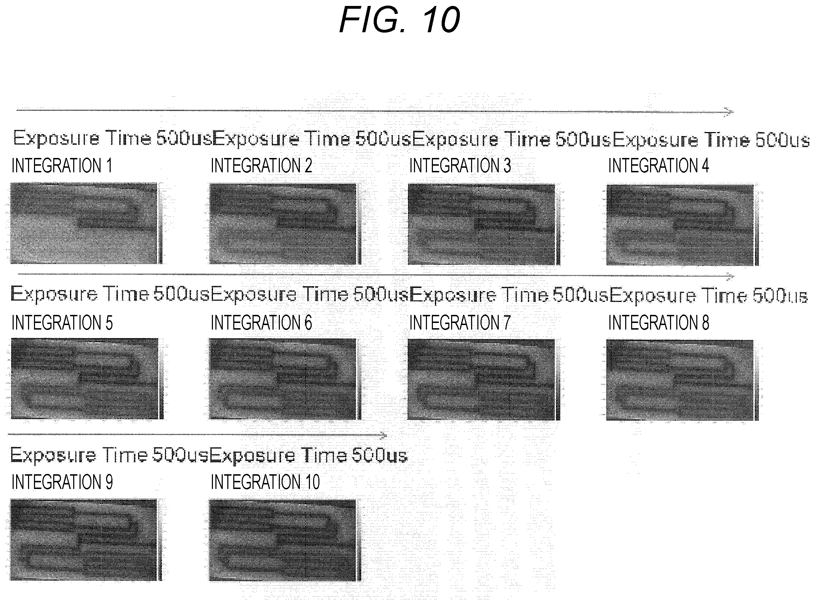

In a second experimental example, blood was fed to a phantom of a living body, uniformly illuminated by a coherent laser light source with a wavelength of 820 nm, and the phantom was imaged at a frame rate of 30 fps and an exposure duration of 500 μs using a SONY global shutter CMOS imager. Speckle images in the number of one (comparative example), two,..., 10 in time series were integrated to obtain speckle integrated images corresponding to the integrated exposure durations of 500 μs (comparative example), 1 ms,..., and 5 ms (refer to Fig. 10). Subsequently, an optimum speckle integrated image from among the speckle integrated images illustrated in Fig. 10 was able to be selected in accordance with the measurement target of the blood flow rate.

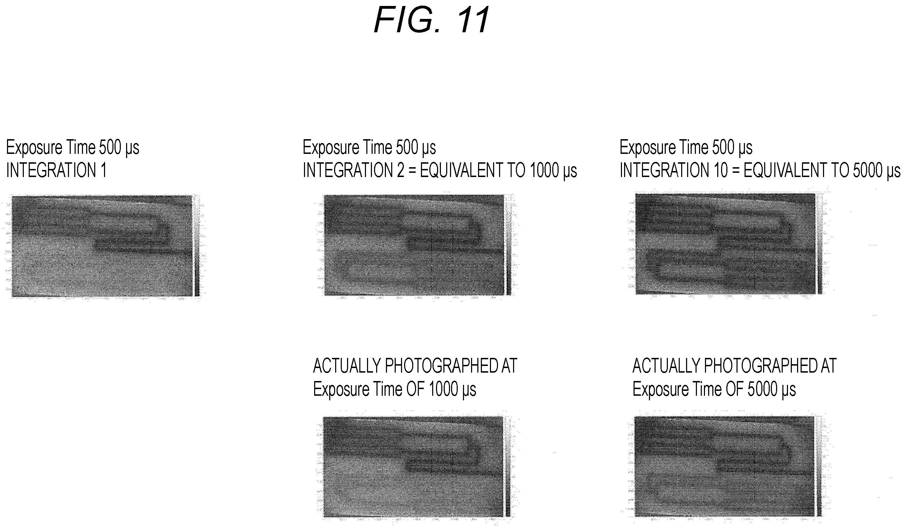

The speckle contrast K depends on the moving speed for individual areas as photographing targets. In the second example, the values of speckle contrast K are set to about 0.6, 0.5, and 0.4, respectively in acquisition of integrated exposure durations of 500 μs, 1 ms, and 5 ms in the case of the blood flow rate of 1 mm/sec. Fig. 11 illustrates each of speckle integrated images corresponding to the integrated exposure duration of 500 μs, 1 ms, 5 ms and the images captured at the actual exposure duration of 1 ms and 5 ms. As illustrated in Fig. 11, it was found that it is possible to obtain each of the speckle integrated images corresponding to the integrated exposure duration of 1 ms and 5 ms that is substantially equivalent images to the images captured at the actual exposure duration of 1 ms and 5 ms.

REFERENCE SIGNS LIST

| 1 | Information processing apparatus |
| 10 | Speckle imaging system |
| 11 | Luminance integrator |
| 12 | Contrast calculation unit |

| 13 | Fluid velocity calculation unit |
| --- | --- |
| 14 | Light source |
| 15 | Imaging apparatus |
| 16 | Display apparatus |
| 17 | Storage apparatus |
| 101 | CPU |
| 102 | ROM |
| 103 | RAM |
| 104 | Bridge |
| 105 | Bus |
| 106 | Interface |
| 107 | Input apparatus |
| 108 | Output apparatus |
| 109 | Storage |
| 110 | Connection port |
| 111 | Communication apparatus |

**Claims**

1. An information processing apparatus (1) comprising:

   a luminance integrator (11) that integrates a luminance of a plurality of speckle images obtained by an imaging element by a plurality of times of imaging of scattered light obtained from an imaging target to which coherent light is emitted; **characterized by**
   a contrast calculation unit (12) that calculates a contrast of a speckle pattern on the basis of a speckle integrated image integrated by the luminance integrator (11) ;
   wherein the contrast calculation unit (12) calculates a variance value and an average value of the luminance in a local image region of the speckle integrated image, and calculates the contrast of the speckle pattern on the basis of the obtained variance value and average value of the luminance.

2. The information processing apparatus according to claim 1, further comprising a fluid velocity calculation unit (13) that calculates a fluid velocity of the imaging target on the basis of an integrated exposure duration obtained by integrating exposure durations of the plurality of speckle images and the contrast of the speckle pattern calculated by the contrast calculation unit (12).

3. The information processing apparatus according to claim 1, wherein the plurality of speckle images is images captured for an exposure duration of 10 ms or less.

4. The information processing apparatus according to claim 1,
   wherein the contrast calculation unit (12) calculates a standard deviation of the luminance by taking a square root of the obtained variance value of the luminance and substitutes the obtained standard deviation and the average value of the luminance into Formula (1) indicating a relationship between the contrast of the speckle pattern, the standard deviation of the luminance, and the average value of the luminance so as to calculate the contrast of the speckle pattern,
   [Mathematical Expression 1]

   $$K = \sigma/\langle I \rangle \quad \cdots \quad (1)$$

   where K is the contrast of the speckle pattern, $\sigma$ is the standard deviation of luminance I, and $\langle I \rangle$ is the average value of luminance I.

5. The information processing apparatus according to claim 2,
   wherein the fluid velocity calculation unit (13) obtains a correlation time on the basis of the integrated exposure duration and the contrast of the speckle pattern, and compares the obtained correlation time with a predetermined correlation time to calculate the fluid velocity.

**6.** The information processing apparatus according to claim 5,
wherein the fluid velocity calculation unit (13) substitutes the integrated exposure duration and the contrast of the speckle pattern into Formula (2) indicating a relationship between the exposure duration, the contrast of the speckle pattern, and the correlation time to obtain the correlation time,
[Mathematical Expression 2]

$$K(T,\tau_c) = \left( \frac{1-e^{-2\frac{T}{\tau_c}}}{2\frac{T}{\tau_c}} \right)^{\frac{1}{2}} \quad \cdots \quad (2)$$

where K $(T, \tau_c)$ is the contrast of the speckle pattern, T is the exposure duration, and $\tau_c$ is the correlation time.

**7.** The information processing apparatus according to claim 5,
wherein the fluid velocity calculation unit (13) substitutes the integrated exposure duration and the contrast of the speckle pattern into Formula (3) indicating the relationship between the exposure duration, the contrast of the speckle pattern, and the correlation time to obtain the correlation time,
[Mathematical Expression 3]

$$K(T,\tau_c) = \left( \beta \frac{e^{-2\frac{T}{\tau_c}} - 1 + 2\frac{T}{\tau_c}}{2\left(\frac{T}{\tau_c}\right)^2} \right)^{\frac{1}{2}} \quad \cdots \quad (3)$$

where K $(T, \tau_c)$ is the contrast of the speckle pattern, T is the exposure duration, $\tau_c$ is the correlation time, and $\beta$ is a value determined when a known blood flow rate is plotted.

**8.** The information processing apparatus according to claim 2, further comprising a display control unit that controls a display unit (16) to display an image.

**9.** The information processing apparatus according to claim 8, wherein the display control unit maps the fluid velocity calculated by the fluid velocity calculation unit (13) to further control the display unit (16) to display fluid velocity distribution.

**10.** The information processing apparatus according to claim 2, wherein the fluid velocity is a blood flow rate in a blood vessel.

**11.** A speckle imaging system comprising:

the information processing apparatus (1) according to any one of claims 1 to 10;
a light source (14) that emits coherent light to an imaging target;
an imaging apparatus (15) that performs, using an imaging element, a plurality of times of imaging of scattered light obtained from the imaging target to which the coherent light is emitted, and outputs the plurality of speckle images; and
a display apparatus (16) that displays an image.

**12.** The speckle imaging system according to claim 11, wherein the display apparatus (16) maps the fluid velocity calculated by the fluid velocity calculation unit (13) to further display fluid velocity distribution.

**13.** An information processing method at least comprising:

a luminance integration step (ST101) of integrating a luminance of a plurality of speckle images obtained by an imaging element by a plurality of times of imaging of scattered light obtained from an imaging target to which coherent light is emitted; **characterized by**
a contrast calculation step (ST102) of calculating a contrast of a speckle pattern on the basis of a speckle integrated image integrated in the luminance integration step;
wherein the contrast calculation step calculates a variance value and an average value of the luminance in a local image region of the speckle integrated image, and calculates the contrast of the speckle pattern on the basis of the obtained variance value and average value of the luminance.

**Patentansprüche**

**1.** Informationsverarbeitungseinrichtung (1), die Folgendes umfasst:

einen Luminanzintegrator (11), der eine Luminanz mehrerer Speckle-Bilder integriert, die durch ein Bildgebungselement mittels mehrmaliger Abbildung gestreuten Lichts erhalten werden, das von einem Bildgebungsziel, zu dem kohärentes Licht emittiert wird, erhalten wird; **gekennzeichnet durch**
eine Kontrastberechnungseinheit (12), die einen Kontrast eines Speckle-Musters auf Basis eines integrierten Speckle-Bildes, das durch den Luminanzintegrator (11) integriert wird, berechnet;
wobei die Kontrastberechnungseinheit (12) einen Varianzwert und einen Durchschnittswert der Luminanz in einem lokalen Bildgebiet des integrierten Speckle-Bildes berechnet und den Kontrast des Speckle-Musters auf Basis des erhaltenen Varianzwerts und Durchschnittswerts der Luminanz berechnet.

**2.** Informationsverarbeitungseinrichtung nach Anspruch 1, ferner umfassend eine Fluidgeschwindigkeitsberechnungseinheit (13), die eine Fluidgeschwindigkeit des Bildgebungsziels auf Basis einer integrierten Belichtungsdauer, die durch Integrieren von Belichtungsdauern der mehreren Speckle-Bilder erhalten wird, und des durch die Kontrastberechnungseinheit (12) berechneten Kontrasts des Speckle-Musters berechnet.

**3.** Informationsverarbeitungseinrichtung nach Anspruch 1, wobei die mehreren Speckle-Bilder Bilder sind, die für eine Belichtungsdauer von 10 ms oder weniger erfasst werden.

**4.** Informationsverarbeitungseinrichtung nach Anspruch 1,
wobei die Kontrastberechnungseinheit (12) eine Standardabweichung der Luminanz durch Nehmen einer Quadratwurzel des erhaltenen Varianzwerts der Luminanz berechnet und die erhaltene Standardabweichung und den Durchschnittswert der Luminanz in Formel (1) einsetzt,
die eine Beziehung zwischen dem Kontrast des Speckle-Musters, der Standardabweichung der Luminanz und dem Durchschnittswert der Luminanz angibt, um den Kontrast des Speckle-Musters zu berechnen,
[Mathematischer Ausdruck 1]

$$K = \sigma / <I> \quad \ldots \quad (1)$$

wobei K der Kontrast des Speckle-Musters ist, $\sigma$ die Standardabweichung der Luminanz I ist und <I> der Durchschnittswert der Luminanz I ist.

**5.** Informationsverarbeitungseinrichtung nach Anspruch 2,
wobei die Fluidgeschwindigkeitsberechnungseinheit (13) eine Korrelationszeit auf Basis der integrierten Belichtungsdauer und des Kontrasts des Speckle-Musters erhält und die erhaltene Korrelationszeit mit einer vorbestimmten Korrelationszeit vergleicht, um die Fluidgeschwindigkeit zu berechnen.

**6.** Informationsverarbeitungseinrichtung nach Anspruch 5,
wobei die Fluidgeschwindigkeitsberechnungseinheit (13) die integrierte Belichtungsdauer und den Kontrast des Speckle-Musters in Formel (2) einsetzt, die eine Beziehung zwischen der Belichtungsdauer, dem Kontrast des Speckle-Musters und der Korrelationszeit angibt, um die Korrelationszeit zu erhalten,
[Mathematischer Ausdruck 2]

$$K(T,\tau_c) = \left( \frac{1-e^{-2\frac{T}{\tau_c}}}{2\frac{T}{\tau_c}} \right)^{\frac{1}{2}} \quad ...\,(2)$$

wobei $K(T,\tau_c)$ der Kontrast des Speckle-Musters ist, T die Belichtungsdauer ist und $\tau_c$ die Korrelationszeit ist.

**7.** Informationsverarbeitungseinrichtung nach Anspruch 5,
wobei die Fluidgeschwindigkeitsberechnungseinheit (13) die integrierte Belichtungsdauer und den Kontrast des Speckle-Musters in Formel (3) einsetzt, die die Beziehung zwischen der Belichtungsdauer, dem Kontrast des Speckle-Musters und der Korrelationszeit angibt, um die Korrelationszeit zu erhalten,
[Mathematischer Ausdruck 3]

$$K(T,\tau_c) = \left( \beta \frac{e^{-2\frac{T}{\tau_c}}-1+2\frac{T}{\tau_c}}{2\left(\frac{T}{\tau_c}\right)^2} \right)^{\frac{1}{2}} \quad ...\,(3)$$

wobei $K(T,\tau_c)$ der Kontrast des Speckle-Musters ist, T die Belichtungsdauer ist, $\tau_c$ die Korrelationszeit ist und $\beta$ ein Wert ist, der bestimmt wird, wenn eine bekannte Blutflussrate grafisch dargestellt wird.

**8.** Informationsverarbeitungseinrichtung nach Anspruch 2, ferner umfassend eine Anzeigesteuereinheit, die eine Anzeigeeinheit (16) zum Anzeigen eines Bildes steuert.

**9.** Informationsverarbeitungseinrichtung nach Anspruch 8, wobei die Anzeigesteuereinheit die durch die Fluidgeschwindigkeitsberechnungseinheit (13) berechnete Fluidgeschwindigkeit abbildet, um die Anzeigeeinheit (16) ferner zum Anzeigen einer Fluidgeschwindigkeitsverteilung zu steuern.

**10.** Informationsverarbeitungseinrichtung nach Anspruch 2, wobei die Fluidgeschwindigkeit eine Blutflussrate in einem Blutgefäß ist.

**11.** Speckle-Bildgebungssystem, das Folgendes umfasst:

die Informationsverarbeitungseinrichtung (1) nach einem der Ansprüche 1 bis 10;
eine Lichtquelle (14), die kohärentes Licht zu einem Bildgebungsziel emittiert;
eine Bildgebungseinrichtung (15), die unter Verwendung eines Bildgebungselements eine mehrmalige Abbildung gestreuten Lichts durchführt, das von dem Bildgebungsziel, zu dem das kohärente Licht emittiert wird, erhalten wird, und die mehreren Speckle-Bilder ausgibt; und
eine Anzeigeeinrichtung (16), die ein Bild anzeigt.

**12.** Speckle-Bildgebungssystem nach Anspruch 11, wobei die Anzeigeeinrichtung (16) die durch die Fluidgeschwindigkeitsberechnungseinheit (13) berechnete Fluidgeschwindigkeit abbildet, um ferner eine Fluidgeschwindigkeitsverteilung anzuzeigen.

**13.** Informationsverarbeitungsverfahren, das zumindest Folgendes umfasst:

einen Luminanzintegrationsschritt (ST101) zum Integrieren einer Luminanz mehrerer Speckle-Bilder, die durch ein Bildgebungselement mittels mehrmaliger Abbildung gestreuten Lichts erhalten werden, das von einem Bildgebungsziel, zu dem kohärentes Licht emittiert wird, erhalten wird; **gekennzeichnet durch** einen Kontrastberechnungsschritt (ST102) zum Berechnen eines Kontrasts eines Speckle-Musters auf Basis eines integrierten Speckle-Bildes, das in dem Luminanzintegrationsschritt integriert wird;
wobei der Kontrastberechnungsschritt einen Varianzwert und einen Durchschnittswert der Luminanz in einem lokalen Bildgebiet des integrierten Speckle-Bildes berechnet und den Kontrast des Speckle-Musters auf Basis des erhaltenen Varianzwerts und Durchschnittswerts der Luminanz berechnet.

**Revendications**

1.  Appareil de traitement d'informations (1) comprenant :
    un intégrateur de luminance (11) qui intègre la luminance d'une pluralité d'images de tavelures obtenues par un élément d'imagerie au moyen d'une pluralité d'imageries de la lumière diffusée obtenue à partir d'une cible d'imagerie vers laquelle une lumière cohérente est émise ; **caractérisé par** :

    une unité de calcul de contraste (12) qui calcule le contraste d'un motif de tavelures sur la base d'une image intégrée de tavelures intégrée par l'intégrateur de luminance (11) ;
    où l'unité de calcul de contraste (12) calcule une valeur de variance et une valeur moyenne de la luminance dans une région d'image locale de l'image intégrée de tavelures, et calcule le contraste du motif de tavelures sur la base de la valeur de variance et de la valeur moyenne de la luminance obtenues.

2.  Appareil de traitement d'informations selon la revendication 1, comprenant en outre une unité de calcul de vitesse de fluide (13) qui calcule une vitesse de fluide de la cible d'imagerie sur la base d'une durée d'exposition intégrée obtenue en intégrant les durées d'exposition de la pluralité d'images de tavelures et le contraste du motif de tavelures calculé par l'unité de calcul de contraste (12).

3.  Appareil de traitement d'informations selon la revendication 1, dans lequel la pluralité d'images de tavelures est constituée d'images capturées pendant une durée d'exposition de 10 ms ou moins.

4.  Appareil de traitement d'informations selon la revendication 1, dans lequel l'unité de calcul de contraste (12) calcule un écart-type de la luminance en prenant une racine carrée de la valeur de variance obtenue de la luminance et substitue l'écart-type obtenu et la valeur moyenne de la luminance dans la formule (1) indiquant une relation entre le contraste du motif de tavelures, l'écart-type de la luminance et la valeur moyenne de la luminance afin de calculer le contraste du motif de tavelures,
    [Expression mathématique 1]

$$K = \sigma/<I> \quad \dots \qquad (1)$$

où K est le contraste du motif de tavelures, $\sigma$ est l'écart-type de la luminance I, et <I> est la valeur moyenne de la luminance I.

5.  Appareil de traitement d'informations selon la revendication 2,
    dans lequel l'unité de calcul de vitesse de fluide (13) obtient un temps de corrélation sur la base de la durée d'exposition intégrée et du contraste du motif de tavelures, et compare le temps de corrélation obtenu avec un temps de corrélation prédéterminé pour calculer la vitesse du fluide.

6.  Appareil de traitement d'informations selon la revendication 5,
    dans lequel l'unité de calcul de vitesse de fluide (13) substitue la durée d'exposition intégrée et le contraste du motif de tavelures dans la formule (2) indiquant une relation entre la durée d'exposition, le contraste du motif de tavelures et le temps de corrélation pour obtenir le temps de corrélation,
    [Expression mathématique 2]

$$K(T, \tau_c) = \left(\frac{1-e^{-2\frac{T}{\tau_c}}}{2\frac{T}{\tau_c}}\right)^{1/2} \quad \dots \qquad (2)$$

où K (T, $\tau_c$) est le contraste du motif de tavelures, T est la durée d'exposition, et $\tau_c$ est le temps de corrélation.

7.  Appareil de traitement d'informations selon la revendication 5,
    dans lequel l'unité de calcul de vitesse de fluide (13) substitue la durée d'exposition intégrée et le contraste du motif de tavelures dans la formule (3) indiquant la relation entre la durée d'exposition, le contraste du motif de tavelures et le temps de corrélation pour obtenir le temps de corrélation, [Expression mathématique 3]

$$K(T, \tau_c) = (\beta \frac{e^{-2\frac{T}{\tau_c}} - 1 + 2\frac{T}{\tau_c}}{2(\frac{T}{\tau_c})^2})^{1/2} \quad \dots \quad (3)$$

où K (T, $\tau_c$) est le contraste du motif de tavelures, T est la durée d'exposition, $\tau_c$ est le temps de corrélation, et β est une valeur déterminée lorsqu'un débit sanguin connu est tracé.

**8.** Appareil de traitement d'informations selon la revendication 2, comprenant en outre une unité de commande d'affichage qui commande une unité d'affichage (16) pour afficher une image.

**9.** Appareil de traitement d'informations selon la revendication 8, dans lequel l'unité de commande d'affichage cartographie la vitesse du fluide calculée par l'unité de calcul de la vitesse du fluide (13) pour contrôler en outre l'unité d'affichage (16) pour afficher la distribution de la vitesse du fluide.

**10.** Appareil de traitement d'informations selon la revendication 2, dans lequel la vitesse du fluide est un débit sanguin dans un vaisseau sanguin.

**11.** Système d'imagerie de tavelures comprenant :

l'appareil de traitement d'informations (1) selon l'une quelconque des revendications 1 à 10 ;
une source de lumière (14) qui émet une lumière cohérente vers une cible d'imagerie ;
un appareil d'imagerie (15) qui effectue, en utilisant un élément d'imagerie, une pluralité d'imageries de la lumière diffusée obtenue à partir de la cible d'imagerie vers laquelle la lumière cohérente est émise, et délivre en sortie la pluralité d'images de tavelures ; et
un appareil d'affichage (16) qui affiche une image.

**12.** Système d'imagerie de tavelures selon la revendication 11, dans lequel l'appareil d'affichage (16) cartographie la vitesse du fluide calculée par l'unité de calcul de vitesse de fluide (13) pour afficher en outre la distribution de la vitesse du fluide.

**13.** Procédé de traitement d'informations comprenant au moins :

une étape d'intégration de luminance (ST101) comprenant d'intégrer la luminance d'une pluralité d'images de tavelures obtenues par un élément d'imagerie au moyen d'une pluralité d'imageries de la lumière diffusée obtenue à partir d'une cible d'imagerie vers laquelle une lumière cohérente est émise ;
le procédé étant **caractérisé par** :

une étape de calcul de contraste (ST102) comprenant de calculer le contraste d'un motif de tavelures sur la base d'une image intégrée de tavelures intégrée dans l'étape d'intégration de la luminance ;
où l'étape de calcul de contraste calcule une valeur de variance et une valeur moyenne de la luminance dans une région d'image locale de l'image intégrée de tavelures, et calcule le contraste du motif de tavelures sur la base de la valeur de variance et de la valeur moyenne de la luminance obtenues.

## FIG. 1

SPECKLE IMAGE → 1

11 — LUMINANCE INTEGRATOR

12 — CONTRAST CALCULATION UNIT

13 — FLUID VELOCITY CALCULATION UNIT

# FIG. 2

# FIG. 3

```
          ┌──────────────┐
          │    START     │
          └──────┬───────┘
                 │
                 ▼
  ST101 ──┌──────────────────────┐
          │  INTEGRATE LUMINANCE │
          └──────────┬───────────┘
                     │
                     ▼
  ST102 ──┌──────────────────────┐
          │  CALCULATE CONTRAST K│
          └──────────┬───────────┘
                     │
                     ▼
  ST103 ──┌──────────────────────┐
          │ CALCULATE CORRELATION│
          │      TIME τc         │
          └──────────┬───────────┘
                     │
                     ▼
  ST104 ──┌──────────────────────┐
          │CALCULATE FLUID VELOCITY│
          └──────────┬───────────┘
                     │
                     ▼
          ┌──────────────┐
          │     END      │
          └──────────────┘
```

# FIG. 4

(A)

(B)

# FIG. 5

t = t

EXPOSURE DURATION 10 ms

t = t     t = t + Δt

+

EXPOSURE DURATION 20 ms

# FIG. 6

START

ST201 — SET EXPOSURE DURATION AND FRAME RATE

ST202 — DETERMINE NUMBER OF IMAGES FOR INTEGRATION

ST203 — CAPTURE SPECKLE IMAGE

ST204 — STORE SPECKLE IMAGE

ST205 — DISPLAY SPECKLE IMAGE

ST206 — INTEGRATE LUMINANCE

ST207 — CALCULATE CONTRAST K

ST208 — CALCULATE CORRELATION TIME $\tau_c$

ST209 — CALCULATE FLUID VELOCITY

ST210 — STORE DISTRIBUTION

ST211 — DISPLAY DISTRIBUTION

END

## FIG. 7

```
                    START

ST301 ──── SET EXPOSURE DURATION

ST302 ──── DETERMINE NUMBER OF IMAGES
            FOR INTEGRATION

ST303 ──── CAPTURE SPECKLE IMAGE

ST304 ──── STORE SPECKLE IMAGE

ST305 ──── DISPLAY SPECKLE IMAGE

ST306 ──── HAS TARGET NUMBER OF        No
            INTEGRATION BEEN REACHED?

                      Yes

ST307 ──── INTEGRATE LUMINANCE

ST308 ──── CALCULATE CONTRAST K

ST309 ──── STORE SPECKLE INTEGRATED IMAGE

ST310 ──── DISPLAY SPECKLE INTEGRATED IMAGE

                    END
```

# FIG. 8

# FIG. 9

LOW FLOW RATE = GRAY

HIGH FLOW RATE = WHITE

STOP = BLACK

FLOW RATE STOP = DARK GRAY

# FIG. 10

Exposure Time 500usExposure Time 500usExposure Time 500usExposure Time 500us

INTEGRATION 1       INTEGRATION 2       INTEGRATION 3       INTEGRATION 4

Exposure Time 500usExposure Time 500usExposure Time 500usExposure Time 500us

INTEGRATION 5       INTEGRATION 6       INTEGRATION 7       INTEGRATION 8

Exposure Time 500usExposure Time 500us

INTEGRATION 9       INTEGRATION 10

EP 3 385 723 B1

*FIG. 11*

Exposure Time 500 μs
INTEGRATION 1

Exposure Time 500 μs
INTEGRATION 2 = EQUIVALENT TO 1000 μs

ACTUALLY PHOTOGRAPHED AT
Exposure Time OF 1000 μs

Exposure Time 500 μs
INTEGRATION 10 = EQUIVALENT TO 5000 μs

ACTUALLY PHOTOGRAPHED AT
Exposure Time OF 5000 μs

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ASHWIN B. PATHARATHY et al.** Robust flow measurement with multi-exposure speckle imaging. *Optics Express,* 2008